(19) Europäisches Patentamt
European Patent Office
Office européen des brevets

(11) Veröffentlichungsnummer: **0 063 692**
**A2**

(12) **EUROPÄISCHE PATENTANMELDUNG**

(21) Anmeldenummer: **82102198.7**

(22) Anmeldetag: **18.03.82**

(51) Int. Cl.³: **C 07 J 19/00**, C 07 J 9/00, C 07 J 17/00, C 07 J 43/00

(30) Priorität: **23.03.81 DE 3111367**

(43) Veröffentlichungstag der Anmeldung: **03.11.82 Patentblatt 82/44**

(84) Benannte Vertragsstaaten: **AT CH DE FR LI**

(71) Anmelder: **HOECHST AKTIENGESELLSCHAFT, Postfach 80 03 20, D-6230 Frankfurt/Main 80 (DE)**

(72) Erfinder: **Welzel, Peter, Prof. Dr. Ruhr-Universität Bochum, Abteilung für Chemie Postfach 10 21 48, D-4630 Bochum 1 (DE)**
Erfinder: **Stein, Hermann, Hochstrasse 33, D-4390 Gladbeck (DE)**
Erfinder: **Milkova, Tsenka, Dr., Kollegstrasse 2, D-4630 Bochum (DE)**

(54) **Verfahren zur Herstellung von Cardenoliden.**

(57) Die Erfindung betrifft ein Verfahren zur Herstellung von Cardenoliden aus einem 20-Methylen-pregnan-21-al durch Umsetzung mit Methoxymethylen-triarylphosphoran zum Gemisch der isomeren 20-Methylen-pregnan-21-methoxymethylene, Anlagerung von Singulett-Sauerstoff zum entsprechenden 1,2-Dioxan und basisch katalysierte Umlagerung des Dioxanrings zum Butenolidring und ein Verfahren zur Herstellung des Digoxigenins oder 12-Epidigoxigenins ausgehend von Desoxycholsäure. Die Erfindung betrifft weiterhin Verbindungen der Formel

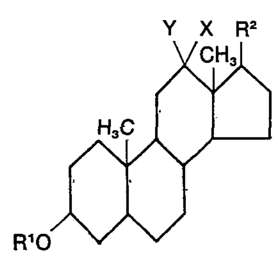

in der R² 5-Oxo-2,5-dihydrofuran-3-yl (I) oder 6-Methoxy-3,6-dihydro-1,2-dioxin-4-yl (II) ist, R¹ für Acyl oder, im Falle R² = I für Acyl oder Wasserstoff steht und X Wasserstoff und Y α-Hydroxy oder X und Y, im Falle R² = II zusammen Oxo bedeuten.

EP 0 063 692 A2

Verfahren zur Herstellung von Cardenoliden

Gegenstand der Erfindung ist ein Verfahren zur Herstellung von Cardenoliden, das insbesondere die Möglichkeit schafft, diese Verbindungsklasse ausgehend von der Desoxycholsäure zu synthetisieren.

Es sind bisher nur wenige Partialsynthesen auf der Grundlage von gut zugänglichen natürlichen Steroid-Vorläufern für 14ß-Hydroxyl-cardenolid-aglukone bekannt geworden, die die entscheidenden für die herzwirksame Wirkung (positiv inotrope Wirkung) essentiellen Strukturmerkmale, einen 17ß-konfigurierten Butenolidring sowie eine 14ß-orientierte Hydroxylgruppe, enthalten. Die Schwierigkeiten und Problemstellungen zur Synthese derartiger 14ß-Hydroxy-cardenolid-aglukone sind von F. Sondheimer (Chem. Brit. 454 (1965)) im einzelnen aufgezählt.

Alle bisher unter diesem Gesichtspunkt erfolgreich synthetisierten 14ß-Hydroxy-cardenolid-aglukone (Digitoxigenin: J ACS 84, 875 (1962); Periplogenin: TL 2045 (1963) und J OC 39, 2319 (1974); Xysmalogenin: Chem. Pharm. Bull., 20, 1585 (1972); Uzarigenin: Liebigs Ann. Chem. 726, 136 (1969); Canarigenin: ibid 727, 110 (1969); Strophanthidin: J OC 43, 3946 (1978)) enthalten zwar in jedem Fall die eben näher angeführten essentiellen Strukturmerkmale, eine 14ß-Hydroxylgruppe sowie einen 17ß-Butenolidring, jedoch nicht die bei den marktgängigen Cardenoliden der Digoxin-Reihe (z.B. Digoxin), für eine optimale Wirkungsdauer und Metabolisierung determinierende 12-ß-Hydroxylgruppe.

Im vorliegenden Fall wird erstmals eine neuartige und originelle Partialsynthese von in 12-Position eine Hydroxylgruppe (12ß oder 12α) tragenden 14ß-Hydroxy-cardenolid-aglukonen auf der Ausgangsbasis Desoxycholsäure, die in 12α-Position bereits eine Hydroxylgruppe als verwertbare funktionelle Gruppe aufweist, mitgeteilt. Das Ausgangsmaterial Desoxycholsäure ist aus den natürlichen Gallensäuren leicht zugänglich.

Das erfindungsgemäße Verfahren zur Herstellung von Cardenoliden ist dadurch gekennzeichnet, daß man ein 20-Methylen-pregnan-21-al mit einem Methoxymethylen-triarylphosphoran, vorzugsweise Methoxymethylen-triphenylphosphoran zum Gemisch der isomeren 20-Methylen-pregnan-21-methoxymethylene umsetzt, an diese Singulett-Sauerstoff zum entsprechenden 1,2-Dioxan anlagert und in diesem mit einer organischen Base den Dioxanring zum Butenolidring umlagert.

Das 20-Methylen-pregnan-21-al kann gemäß einer weiter unten beschriebenen Reaktionsfolge hergestellt werden. Die Umsetzung mit Methoxymethylen-triarylphosphoran ist für ähnliche Verbindungen als "Wittig-Reaktion" beschrieben.

Die Umsetzung mit Singulett-Sauerstoff erfolgt im Wege einer (4+2)-Cycloaddition unter Bildung eines 1,2-Dioxanringes, der anschließend mit Hilfe einer organischen Base in den entsprechenden Butenolid-Ring umgelagert wird. Als organische Base eignen sich hier vorzugsweise tertiäre organische Basen beispielsweise Triethylamin.

Das erfindungsgemäße Verfahren zur Herstellung des Digoxigenins oder 12-Epidi-digoxigenins ist dadurch gekennzeichnet, daß man ein 3ß-Acetoxy-12-oxo-5-ß-14-α-card-20(22)-enolid (13) durch Photolyse in den ungesättigten Aldehyd (14) umwandelt, diesen einer intramolekularen Prins-Reaktion unterwirft, wobei die stereoisomeren 12,14-ß-Diole (16a) und (16b) entstehen, und gegebenenfalls aus diesen die Acetylgruppe in 3-Stellung verseift.

Die beschriebenen Umsetzungen sind durch die Formelbilder auf den Seiten 17 und 18 veranschaulicht. In diesen Formeln und im Text bedeutet Ac Acyl, also einen Carboxylsäurerest, vorzugsweise Acetyl.

Die Photolyse von (13) ergibt durch Norrish-I-Spaltung den ungesättigten Aldehyd (14), der unter den Reaktionsbedingungen teilweise in einer intramolekularen Paterno-Büchi-Reaktion zum Oxetan (15) weiterreagiert. In schwach saurer

Lösung isomerisiert das Oxetan (15) zum Aldehyd (14) zurück, der in einer intramolekularen Prins-Reaktion ein Gemisch der beiden 12-stereoisomeren 12,14-ß-Diole (16a) und (16b) ergibt. Die Abspaltung der Estergruppen an $C_3$ und damit Freisetzung von Digoxigenin (17a) und 12-Epidigoxigenin (17b) gelingt vorzugsweise unter sauren Bedingungen.

Das 3ß-Acetoxy-12-oxo-5-ß-14α-card-20(22)-enolid (13) kann ausgehend von der Desoxycholsäure (1) wie folgt erhalten werden: Desoxycholsäure (1) wird mit Carbonyldiimidazol in das Imidazolid (2) übergeführt und dieses nach Iwasaki photochemisch zur 20-Methylenverbindung (3) abgebaut. Aus (3) wird (4) durch Konfigurationsumkehr an $C_3$ nach dem Mitsunobu-Verfahren hergestellt. Die Acylierung von (4) ergibt (5), das mit m-Chlorperbenzoesäure zu einem 8:1 Gemisch der Stereoisomeren der Oxide (6) umgesetzt wird. Umlagerung der Epoxide in eine Allylalkoholgruppierung gelingt mit Aluminiumisopropylat als Base; gleichzeitig tritt Esterhydrolyse ein. Der dabei gebildete Allylalkohol (7) wird zum ungesättigten Aldehyd (8) oxidiert, wofür insbesondere Braunstein geeignet ist, und dieser wird in einer Wittig-Reaktion mit Methoxymethylentriphenylphosphoran zum Gemisch der isomeren Dienoläther (9) umgesetzt. Die Dienoläther (9) reagieren mit Singulett-Sauerstoff in einer (4+2) Cycloaddition zu den 1,2-Dioxanen (10) wobei ein Gemisch der 23-Isomeren entsteht. Aus diesen wird mit einer organischen Base, z.B. Triethylamin, durch heterolytische Öffnung der Peroxidgruppierung und Lactonringschluß das 14α-Cardenolid (11) gebildet werden. Zur Einführung der 14ß-OH-Gruppe wird (11) durch selektive Acylierung an $C_3$ und Oxidation an $C_{12}$ in das 12-Keton (13) umgewandelt.

Die Darstellung des 3ß-Acetoxy-12α-hydroxy-5ß,14α-cardenolids (12) kann vereinfacht werden, in dem man (4) mit Singulett-Sauerstoff oxidiert, wobei ein Gemisch der 3-Acyl-Verbindungen 18 und 19 resultiert. Das darin vorhandene 18 kann abgetrennt oder, beispielsweise mit Braunstein, ebenfalls zu 19 oxidiert werden. 19 läßt sich dann, ohne

daß die Zwischenstufen isoliert werden, durch Reaktion mit Methoxymethylentriphenylphosphoran anschließender Cycloaddition mit Singulett-Sauerstoff und Umlagerung mit Triäthylamin direkt in 12 überführen.

Das gemäß der Erfindung erhaltene Verfahrensprodukt stellt aufgrund der Reaktionsführung 13 ⟶(14)⟶16, bei der regioselektiv die essentielle 14ß-Hydroxylgruppe eingeführt wird, ein Gemisch von 12-Hydroxycardenolid-aglukonen mit 12ß-Hydroxylgruppen (= Digoxigenin = Aglukon des therepeutisch bedeutungsvollen Digoxins) und 12α-Hydroxylgruppen (= etwa esterem gleich stark wirksameren und auch aus ersterem nach Pharm. Acta. Helv. 45, 261 (1970) herstellbarem 12-Epi-digoxigenin) dar, die in Form ihrer 3-Acetate getrennt werden können oder in Form des Gemisches zu dem 12-Dehydro-digoxigenin (einheitlich), das ebenfalls aktuelle Herzwirksamkeit aufweist, oxydiert werden kann.

Die verschiedenen in 12-Stellung eine 12ß- oder 12α-Hydroxylgruppe oder eine 12-Ketogruppe tragenden synthetischen 14ß-Hydroxy-cardenolid-aglukone können nach an sich bekannten Verfahren in ihre in 3-Stellung glykosidierten 3-Glykoside (z.B. Rhamnoside, Gluoside, Digitoxoside etc.) überführt werden und pharmakologisch oder therapeutisch Verwendung finden.

Beispiel 1:

Abbau von Desoxycholsäure (1) zu 20-Methylen-5ß-pregnan-3α,12α-diol (3):

4.95 g (12.5 mMol) 1 (getrocknet) und 2.15 g (13 mMol) N,N'-Carbonyldiimidazol wurden in 50 ml absol. Tetrahydrofuran 3 h bei Raumtemperatur gerührt. Dann wurden nochmals 200 mg N,N'-Carbonyldiimidazol zugesetzt.
Im DC war die Bildung des polaren 2 erkennbar (DC: Aceton, 1x). Ohne weitere Aufarbeitung wurde mit absol. Tetrahydrofuran auf 500 ml verdünnt und in einer Umlaufapparatur mit fallendem Flüssigkeitsfilm (DEMA UV 13/61 mit Quarzfilter) 24 h unter Argon bei Raumtemperatur mit einer Hg-Hochdrucklampe (Philips HPK-125) bestrahlt.
Anschließend wurden zur Zersetzung von noch vorhandenem N,N'-Carbonyldiimidazol 1.5 ml dest. Wasser zugesetzt und danach wurde das Lösungsmittel i.V. abgezogen.
2 derartige Photolyseansätze wurden vereinigt und an 380 g Kieselgel (Petrolether/Aceton = 4:1) aufgetrennt.
Erhalten wurden 3.5 g amorphes 3 (57.8 % bezogen auf umgesetztes 1); zurückgewonnen wurden 2.7 g (27 %) 1.

IR ($CCl_4$): 3600 (OH, frei); 3500 - 3100 (max. bei 3450, CH, gebunden); 1640 $cm^{-1}$ (C=C)

$^1H$-NMR ($CDCl_3$): $\delta$ = 4.88, 4.77 (je ein br. s, $CH_2$-22), 3.90 (m, $W_{1/2}$ = 6 Hz, 12 ß-H), 4.00 - 3.10 (m, 3 ß-H), 3.00 - 2.48 (m, 17α-H), 1.83 (s, $CH_3$-21), 1.00 (s, $CH_3$-19), 0.67 (s, $CH_3$-18)

MS: m/z (rel. Int.) = 332 (28 %), 314 (100 %), 299 (20 %), 296 (24 %), 281 (20 %).

Beispiel 2:

20-Methylen-5ß-pregnan-3ß,12α-diol-3-acetat (4):

1.15 g (3.5 mMol) 3, 0.21 g (3.5 mMol) Essigsäure und 1,31 g (5 mMol) Triphenylphosphin in 15 ml absol. Tetrahydrofuran wurden unter Rühren bei Raumtemperatur mit 0.87 g (5 mMol) Diethylazodicarboxylat in 5 ml Tetrahydrofuran tropfenweise versetzt. Nach Abklingen der Wärmeentwicklung wurde 70 h bei Raumtemperatur gerührt. Abdampfen des Lösungsmittels und Auftrennung des gelben Rückstandes an 150 g Kieselgel (Petrolether(Essigester = 8:1) ergab 941.5 mg (73 %) 4.

Schmp. 167°C (aus Aceton/$H_2O$)

IR ($CHCl_3$): 3600 - 3350 (max. bei 3450, OH), 1720 (C=O), 1640 (C=C), 1260 $cm^{-1}$ (C-O)

$^1$H-NMR ($CDCl_3$): $\delta$ = 5.05 (m, $W_{1/2}$ = 8 Hz, 3α-H), 4.85 und 4.73 (je 1 br. s, $CH_2$-22), 3.81 (m, $W_{1/2}$= 10 Hz, 12 ß-H), 2.70 - 2.30 (m, 17α-H), 2.00 (s, 3ß-OAc), 1.68 (s, $CH_3$-21), 0.93 (s, $CH_3$-19), 0.57 (s, $CH_3$-18)

MS: m/z (rel.Int.) = 374 (15 %), 356 (55 %), 341 (7 %), 314 (13 %), 296 (13 %), 285 (20 %), 281 (12 %), 134 (100 %).

Beispiel 3:

20-Methylen-5ß-pregnan-3ß,12α-diol-diacetat (5):

Zu 374 mg (1 mMol) 4 und 5 mg 4-Dimethylaminopyridin in 4 ml Pyridin wurden 204 mg (2 mMol) Acetanhydrid in 1 ml Pyridin bei Raumtemperatur getropft; anschließend wurde 16 h gerührt. Pyridin und überschüssiges Anhydrid wurden weitgehend i.V. abgezogen. Der Rückstand wurde an 5 g Kieselgel (Petrolether/Essigester = 2:1) getrennt; Ausbeute an 5 : 410 mg (99 %).

Schmp. 128°C (aus Aceton/$H_2O$)

IR ($CCl_4$): 1730 (C=O), 1640 (C=C), 1240 $cm^{-1}$ (C-O)

$^1$H-NMR ($CDCl_3$): $\delta$ = 5.00 (m, 3 -H), 4.87 (m, 12ß-H), 4.82 und 4.50 (je 1 br. s, $CH_2$-22), 2.80 - 2.30 (s, 3ß-OAc), 1.58 (br. s, $CH_3$-21), 0.93 (s, $CH_3$-19), 0.62 (s, $CH_3$-18)

MS: m/z (rel.Int.) = 416 (3 %), 374 (7 %), 356 (48 %), 341 (6 %), 328 (2 %), 313 (9 %), 312 (3 %), 296 (26 %), 281 (19 %), 134 (100 %).

Beispiel 4:
(20-R,S)-20,21 Epoxy-20-methyl-5ß-pregnan-3ß,12α-diol-diacetat (6):

400 mg (0.96 mMol) 5 und 250 mg (1.2 mMol) m-Chlorperbenzoesäure (85 proz.) wurden in 10 ml absol. Methylenchlorid 2 h bei Raumtemperatur gerührt.
Dann wurde einmal mit 10 ml gesättigter $Na_2S_2O_3$-Lösung und je dreimal mit je 10 ml 5 proz. $NaHCO_3$-Lösung und mit $H_2O$ gewaschen. Die wässrigen Phasen wurden einmal mit Methylenchlorid extrahiert. Die vereinigten organischen Phasen wurden über $Na_2SO_4$ getrocknet und nach Filtration eingedampft.
Es wurden 412 mg (99 %) Rohprodukt und nach Umkristallisation aus Petrolether/Essigester 368 mg (89 %) der beiden Diastereomeren 6 erhalten, die chromatographisch nicht getrennt werden konnten. Nach $^1$H-NMR ergibt sich ein Diastereomerenverhältnis von 8:1.

Schmp. 182 - 185°C (aus Aceton/$H_2O$)

IR ($CHCl_3$): 1720 (C=O), 1260 und 1240 $cm^{-1}$ (C-O)

$^1$H-NMR ($CDCl_3$): $\delta$ = 5.00 (m, $W_{1/2}$ = 6 Hz, 3$\alpha$,12ß-H), 2.80 und 2.40 (je 1 m, $CH_2$-21), 2.06 (s, 12 -OAc), 2.00 (s, 3ß-OAc), 1.20 (s, $CH_3$-22), 0.97 und 0.90 (je 1 s, 8:1, $CH_3$-19), 0.77 und 0.60 (je 1 s, 8:1, $CH_3$-18)

MS: m/z (rel.Int.) = 432 (15 %), 372 (100 %), 357 (41 %), 312 (22 %), 297 (17 %)

Beispiel 5:

20-Methylen-5ß-pregnan-3ß, 12 ,21-triol (7):

432 mg (1 mMol) 6 und 430 mg (2 mMol) Aluminiumtriisopropylat wurden in 25 ml absol. Toluol unter Rühren 5 h zum Sieden erhitzt. Dann wurden nochmals 430 mg Aluminiumtriisopropylat zugesetzt, und die Lösung wurde 1.5 h unter Rückfluß erhitzt. Der Ansatz wurde mit Essigester auf 100 ml verdünnt und dreimal mit je 100 ml gesättigter $(NH_4)_2SO_4$-Lösung und mit $H_2O$ gewaschen. Die wässrigen Phasen wurden einmal mit Essigester extrahiert und die vereinigten organischen Phasen über $Na_2SO_4$ getrocknet. Nach Filtration wurde eingedampft.
Ausbeute: 271.4 mg /78 %) 7.

Schmp. 167 - 167.5°C (aus Essigester/Petrolether)

IR ($CHCl_3$): $\delta$ 3600 - 3200 (max. bei 3400, OH), 1640 $cm^{-1}$ (C=C)

$^1$H-NMR ($CDCl_3$): = 5.06 und 4.87 (je 1 m, $W_{1/2}$ = 4 Hz, $CH_2$-22), 4.17 (m, $W_{1/2}$ = 4 Hz, $CH_2$-21), 4.13 (m, $W_{1/2}$ = 6 Hz, 3$\alpha$-H), 3,87 $W_{1/2}$ = 6 Hz, 12ß-H), 3.70 - 3.10 (m, 17$\alpha$-H), 0.95 (s. $CH_3$ - 19), 0.60 (s.$CH_3$-18)

MS: m/z (rel.Int.) = 348 (2 %), 330 (100 %), 315 (30 %), 312 (30 %).

Beispiel 6:

3ß,12α-Dihydroxy-20-methylen-5ß-pregnan-21-al (8):

140 mg (0.40 mMol) 7 und 1.0 g Mangandioxid (aktiviert) wurden in 3 ml absol. Methylenchlorid 1 h bei Raumtemperatur gerührt. Das Reaktionsgemisch wurde dann filtriert, das Filtrat i.V. eingedämpft und der Rückstand an 5 g Kieselgel (Petrolether/Essigester = 3:1, dann 1:1) chromatographisch getrennt.
Ausbeute an 8 : 96.3 mg (69 %)

Schmp. 192 - 193°C (aus Essigester/Petrolether)

IR ($CHCl_3$): 3600 - 3200 (max. bei 3480, OH) 2710 (CHO), 1680 (C=O, ungesättigt), 1620 $cm^{-1}$ (C=C, konjugiert)

$^1$H-NMR ($CDCl_3$): $\delta$ = 9.47 (s, CHO), 6.30 und 6.12 (je 1 br. s, $CH_2$-22), 4.15 (m, $W_{1/2}$ = 6 Hz, 12ß-H), 4.03 (m, $W_{1/2}$ = 6 Hz, 3α-H), 3.27 (m, $W_{1/2}$= 6 Hz, 17α-H), 0.90 (s, $CH_3$-19), 0.51 (s, $CH_3$-18)

MS: m/z (rel.Int.) = 346 (34 %), 331 (2 %), 328 (9 %), 318 (5 %), 313 (100 %).

Beispiel 7:

Umsetzung des Aldehyds 8 mit Methoxymethylentriphenylphosphoniumchlorid und Kalium-tert.-butylat:

123.2 mg (0.36 mMol) Methoxymethylentriphenylphosphoniumchlorid wurden unter Argon in 15 ml absol. Ether suspendiert. Bei -25°C wurden unter Rühren 121.2 mg (1.08 mMol) Kalium-

tert-butylat zugesetzt. Die charakteristische Rotorangefärbung des Ylids entstand innerhalb von 1 Min. Es wurde noch 10 Min. gerührt dann wurden bei -25°C 103.8 mg (0.30 mMol) 8 zugesetzt. Der Reaktionsansatz entfärbte sich allmählich (leicht gelbliche Suspension). Unter Rühren wurde dann innerhalb von 30 Min. auf Raumtemperatur aufgewärmt.
Die Feststoffe wurden durch Zentrifugieren entfernt und zweimal mit Ehter gewaschen. Die vereinigten organischen Phasen wurden bei 25°C i.V. eingedampft und ergaben 101.0 mg mit Triphenylphosphinoxid verunreinigte Dienolether 9.
Die Dienolether 9 sind sehr empfindliche Substanzen, sie wurden deshalb ohne weitere Reinigung umgesetzt. Ihre Bildung wurde durch DC (2 Flecken, polarer als 8, Laufmittel: Petrolether/Essigester = 1:2) und spektroskopisch nachgewiesen.

CD (Cyclohexan): $\lambda_{max}$ = 237 nm

$^1$H-NMR (Pyridin-$d_5$): $\delta$ = 7.40 - 7.10 (aromat. H von $(C_6H_5)_3PO$), 5.85 (m, $W_{1/2}$ = 6 Hz, 23-H), 5.20 - 4.90 (m, $CH_2$-21 und 22-H), 4.30, 4.25, 4.13 und 4.00 (3α-H, 12ß-H, 2x OH), 3.48 und 3.42 (je 1 s, $OCH_3$, 2 Isomere), 3.30 (s, $OCH_3$, Überschuß Reagenz), 3.10 (m, $W_{1/2}$ = 5 Hz, 17α-H), 0.95 (s, $CH_3$-19), 0.65 (s, $CH_3$-18)

MS: m/z (rel.Int.) = 374 (100 %), 356 (30 %), 342 (77 %), 341 (27 %), außerdem 277 (100 %) und 201 (100 %) von $(C_6H_5)_3PO$

Beispiel 8:
Reaktion der Dienolether 9 mit Singulett-Sauerstoff:

Eine Lösung von 25 mg (0.07 mMol) 9 enthaltenden Gemisches (s.o.) und 0.5 mg Bengalrosa B in 5 ml absol. Methylenchlorid, durch die ein trockener, mit Methylenchlorid gesättigter Sauerstoffstrom geleitet wurde, wurde bei 25°C unter Rühren 10 Min. mit einer 1000 W-Halogenlampe bestrahlt. Durch DC (Petrolether/Essigester = 1:2) wurde nachgewiesen, daß die Dienolether zu einem polareren Produkt abreagiert hatten. Nach Eindampfen i.V. bei 25°C zeigte das $^1$H-NMR-Spektrum ($CCl_4$) folgende Signale:

$\delta$ = 7.40 - 7.10 (aromat. H von $(C_6H_5)_3PO$), 5.45 (m, 22-H), 4.90 (m, 23-H), 4.60 - 3.70 (mehrere m, 3α-H, 12ß-H, $CH_2$-21, 2x OH), 4.15 und 4.05 (je 1 s, $OCH_3$, 2 Isomere), 0.90 (s, $CH_3$-19), 0.60 und 0.52 (je 1 s, $CH_3$-18), 2 Isomere)

Beispiel 9:

3ß,12α-Dihydroxy-5ß,14α-card-20 (22)-enolid (11):

Zu 40 mg (0.1 mMol) eines die Dioxane 10 enthaltenden Gemisches (s.o.) in 3 ml absol. Methylenchlorid wurden unter Rühren bei Raumtemperatur 100 mg (1 mMol) Triethylamin zugesetzt, dann wurde noch 5 Min. gerührt. Laut DC (Petrolether/Essigester = 1:2, 1 x ) hatten die Dienolether 10 vollständig zu 11 abreagiert. Der Ansatz wurde zur Trockne eingedampft. Ausbeute: 37 mg.

$^1$H-NMR ($CDCl_3$): $\delta$ = 5.70 (m, $W_{1/2}$ = 3 Hz, 22-H), 4.70 (br. s, $CH_2$-21), 4.00 (m, $W_{1/2}$ = 6 Hz 3α-H), 3.70 (m, $W_{1/2}$ = 5 Hz, 12ß-H), 3.06 (m, $W_{1/2}$ = 3 Hz, 17α-H), 0.93 (s, $CH_3$-19), 0.60 (s, $CH_3$-18)

MS: m/z (rel.Int.) 374 (5 %), 356 (16 %), 346 (6 %), 341 (5 %), 338 (7 %), 328 (100 %), 313 (56 %)

Beispiel 10:

3ß-Acetoxy-12α-hydroxy-5ß,14α-card-20(22)-enolid (12):

49.1 mg (0.13 mMol) 11 und 18.3 mg (0.18 mMol) Acetanhydrid

wurden bei 40°C 10 h gerührt; nach 6 h wurden nochmals 18.5 mg (0.18 mMol) Acetanhydrid zugesetzt. Das Reaktionsgemisch wurde mit einigen Tropfen $H_2O$ versetzt und 1 Min. gerührt; anschließend wurde bei 35°C zur Trockne eingedampft. Der Rückstand wurde säulenchromatographisch an 5 g Kieselgel getrennt (Petrolether/Essigester = 3:1) und ergab 23.2 mg (63.8 %, bezogen auf umgesetztes 11) 12. Es wurden 16.4 mg (33.4 %) 11 zurückgewonnen.
Ausgehend von 8 betrug die Ausbeute an 12 15 %.

IR ($CCl_4$): 3350 - 3200 (max. bei 3470, OH), 1785 und 1745 (Lacton), 1740 (Acetat), 1630 (C=C), 1260 $cm^{-1}$ (C-O)

$^1$H-NMR ($CDCl_3$): $\delta$ = 5.80 (m, $W_{1/2}$ = 6 Hz, 22-H), 5.00 (m, $W_{1/2}$ = 7 Hz, 3α-H), 4.73 (br. s, $CH_2$-21), 3.80 (m, $W_{1/2}$ = 8 Hz, 12ß-H), 3.10 (m, $W_{1/2}$ = 8 Hz, 17α-H), 2.00 (s, 3ß-OAc), 0.92 (s, $CH_3$-19), 0.62 (s, $CH_3$-18)

MS: m/z (rel.Int.)= 416 (6 %), 398 (3 %), 356 (28 %), 341 (6 %), 338 (16 %), 328 (100 %), 327 (52 %), 323 (12 %), 313 (29 %)

## Beispiel 11:

## 3ß-Acetoxy-12-oxo-5ß,14α-card-20(22)-enolid (13):

17.5 mg (0.042 mMol) 12 in 0.5 ml absol. Methylenchlorid gelöst wurden zu 13.6 mg (0.063 mMol) Pyridiniumchlorochromat und 1.8 mg (0.013 mMol) Natriumacetat-trihydrat in 0.5 ml Methylenchlorid unter Rühren zugegeben. Nach 30 Min. wurde mit 2 ml absol. Ether versetzt und 1 h gerührt; anschließend wurde über 0.5 g Kieselgel filtriert und mit Ether eluiert.
Ausbeute: 13.2 mg (77 %) 13.

$^1$H-NMR ($CDCl_3$) $\delta$ = 5.87 (m, $W_{1/2}$ = 3 Hz, 22-H), 5.07 (m,

$W_{1/2}$ = 6 Hz, 3α-H), 4.85 (br. s, $CH_2$-21), 3.03 (t, 17α-H), 2.06 (s, 3ß-OAc), 1.10 (s, $CH_3$-18), 0.92 (s, $CH_3$-19)

MS: m/z (rel.Int.)= 414 (32 %), 399 (17 %), 386 (3 %), 372 (3 %), 370 (1 %), 354 (100 %), 339 (59 %), 336 (59 %), 326 (10 %), 311 (27 %)

Beispiel 12

Digoxigenin-3-acetat (16a) und 12-Epidigoxigenin-3-acetat (16b):

15.9 mg (0.038 m mol) 13 in 3 ml Methylenchlorid wurden unter Durchleiten von Argon bei Raumtemperatur 30 Minuten mit einer Hg-Hochdrucklampe (Philips HPK 125) durch ein Pyrexfilter bestrahlt. Nach Eindampfen wurden die Photolyseprodukte (14 und 15) in 1 ml Tetrahydrofuran gelöst, mit 2 ml eines Gemisches von Essigsäure/Trifluoressigsäure/Wasser = 2.5:1:1 versetzt und bei 15°C 24 h gerührt. Bei Raumtemperatur wurde zur Trockne eingedampft. Der Rückstand wurde säulenchromatographisch an 3 g Kieselgel aufgetrennt (Petrolether/Essigester = 3:2, danach 2:3).
Ausbeute an 16a : 4.6 mg (28 %)
Ausbeute an 16b : 7.2 mg (44 %).
Die Cardenolide 16a und 16b waren identisch mit auf unabhängigem Wege hergestellten Vergleichsproben.

Beispiel 13:

Digoxigenin (17a) und 12-Epidigoxigenin (17b):

9.6 mg (0.02 mMol) 16a wurden in 0.5 ml 1.5 %iger methanolischer HCL 16 h bei Raumtemperatur gerührt. Anschließend wurde bei 25°C eingedampft, mit dest. Wasser versetzt und mit Essigester extrahiert. Nach Trocknen der organischen Phase über $Na_2SO_4$ wurde filtriert und das Filtrat eingedampft.
Ausbeute: 8.2 mg (95 %) 17a
Der Produkt war mit einer authentischen Probe Digoxigenin identisch.
Analog wurde eine Probe 16b gespalten. Das Produkt war identisch mit auf unabhängigem Weg hergestelltem 12-Epidigoxigenin (17b).

Beispiel 14

20-Methylen-5ß-pregnan-3ß,12α,21-triol-3-acetat (18):

Die Lösung von 935 mg (2.5 m mol) 4 und 20 mg Methylenblau B in 80 ml Aceton, durch die ein trockener, mit Aceton gesättigter Sauerstoffstrom geleitet wurde, wurde unter Rühren bei 25°C 11 h mit einer 1000 W-Halogenlampe bestrahlt. Nach Zugabe von 1 g Natriumiodid wurde 1 h bei Raumtemperatur gerührt. Dann wurde die Lösung auf ein Drittel eingeengt, mit 50 ml Essigester versetzt und je dreimal mit $Na_2S_2O_3$-Lösung und mit Wasser (jeweils 50 ml) gewaschen. Nach Trocknen über $Na_2SO_4$ und Filtration wurde i.V. eingedampft. Auftrennung des Rückstandes an 80 g Kieselgel (Petrolether/Essigester = 9:1) ergab 254.4 mg (36 % bezogen auf umgesetztes 4) 18 + 199.7 mg (23.5 %) 19

Wiedergewonnen wurden 258.0 mg (28 %) 4.

Schmp.: 163 - 164°C (aus Aceton/Wasser)

IR ($CCl_4$): 3550 - 3200 (OH), 1730 (C=O), 1240 $cm^{-1}$ (C-O)

$^1$H-NMR ($CDCl_3$): δ = 4.97 (m, $W_{1/2}$ = 5 Hz, 3α-H), 4.97 und 4.77 (je 1 m, $W_{1/2}$ = 5 Hz, $CH_2$-22), 4.10 (br. s, $CH_2$-21), 3.80 (m, $W_{1/2}$ = 8 Hz, 12ß-H), 3.45 (m, $W_{1/2}$ = 15 Hz, 17α-H), 2.00 (s, 3ß-OAc), 0.94 (s, $CH_3$-19), 0.60 (s, $CH_3$-18)

MS: m/z (rel.Int.) = 372 (33 %), 354 (14 %), 339 (2 %), 330 (7 %), 312 (38 %), 297 (15 %), 288 (13 %), 107 (100 %)

Beispiel 15

3ß-Acetoxy-12α-hydroxy-20-methylen-5ß-pregnan-21-al (19):

A) aus 4 :

Bei der Darstellung von 18 wurden 199.7 mg (23.5 % bezogen auf den Umsatz an 4) 19 erhalten.

B) aus 18:

156 mg (0.4 m mol) 18 und 1.50 g Mangandioxid (aktiviert) wurden in 5 ml absol. Methylenchlorid 30 Minuten bei Raumtemperatur gerührt. Nach Filtration wurde das Reaktionsgemisch an 8 g Kieselgel (Methylenchlorid, danach Aceton) chromatographisch getrennt.

Ausbeute an 19 : 137 mg (88 %).

C) aus 8 :

34.6 mg (0.10 m mol) 8, 12 mg (0.12 m mol) Acetanhydrid und 0.5 mg 4-Dimethylaminopyridin in 1 ml Pyridin wurden 3 h bei Raumtemperatur gerührt. Das Lösungsmittel wurde weitgehend i.V. abgezogen. Der Rückstand wurde an 1 g Kieselgel (Petrolether/Essigester = 2:1) getrennt.

Ausbeute an 19 : 37.2 mg (96 %)

Schmp.: 172 - 174°C (aus Petrolether/Essigester)

IR ($CCl_4$): 3550 - 3250 (Max. bei 3470, OH), 2710 (Schulter, CHO), 1720 (Ester), 1680 (C=O, ungesättigt), 1260 (C-O)

$^1$H-NMR ($CCl_4$): $\delta$ = 9.40 (s, CHO), 6.27 und 6.09 (je 1 br. s, $CH_2$-22), 4.93 (m, $W_{1/2}$ = 3 α-H), 3.70 (m, $W_{1/2}$ = 7 Hz, 12ß-H), 3.13 (m, $W_{1/2}$ = 8 Hz, 17α-H), 1.97 (s, 3ß-OAc), 0.90 (s, $CH_3$-19), 0.45 (s, $CH_3$-18)

MS: m/z (rel.Int.) = 388 (35 %), 370 (9%), 360 (4%), 355 (100 %), 328 (13%), 310 (13%), 304 (65%)

Beispiel 16

3ß-Acetoxy-12α-hydroxy-5ß,14α-card-20(22)-enolid (12) aus 19:

41.0 mg (0.12 m mol) Methoxymethylentriphenylphosphoniumchlorid wurden unter Argon in 5 ml absol. Ether suspendiert. Bei -25°C wurden unter Rühren 13.5 mg (0.12 m mol) Kalium-tert-butylat zugesetzt. Es wurde nach Entstehen des Ylids (Rotorangefärbung) noch 10 Minuten gerührt, dann wurden bei -25°C 37.2 mg (0.096 m mol) 19 zugesetzt. Nach Entfärbung wurde 20 Minuten gerührt, dann wurde auf Raumtemperatur

aufgewärmt.

Die Feststoffe wurden durch Zentrifugieren entfernt und zweimal mit Ether gewaschen. Die Etherphasen wurden bei 25°C i.V. eingedampft. Der Rückstand wurde zusammen mit 0.5 mg Bengalrosa B in 5 ml absol. Methylenchlorid gelöst. Durch die Lösung wurde ein trockener, mit Methylenchlorid gesättigter Sauerstoffstrom geleitet und bei 25°C wurde unter Rühren 20 Minuten mit einer 1000 W-Halogenlampe bestrahlt. Nach Zusatz von 100 mg Triethylamin wurde 10 Minuten bei Raumtemperatur gerührt und dann zur Trockene eingedampft. Der Rückstand wurde säulenchromatographisch an 5 g Kieselgel (Petrolether/Essigester = 3:2) aufgetrennt.

Ausbeute an 12: 18.5 mg (57 % bezogen auf umgesetztes 19)
Wiedergewonnen wurden 7.0 mg (19 %) 19.

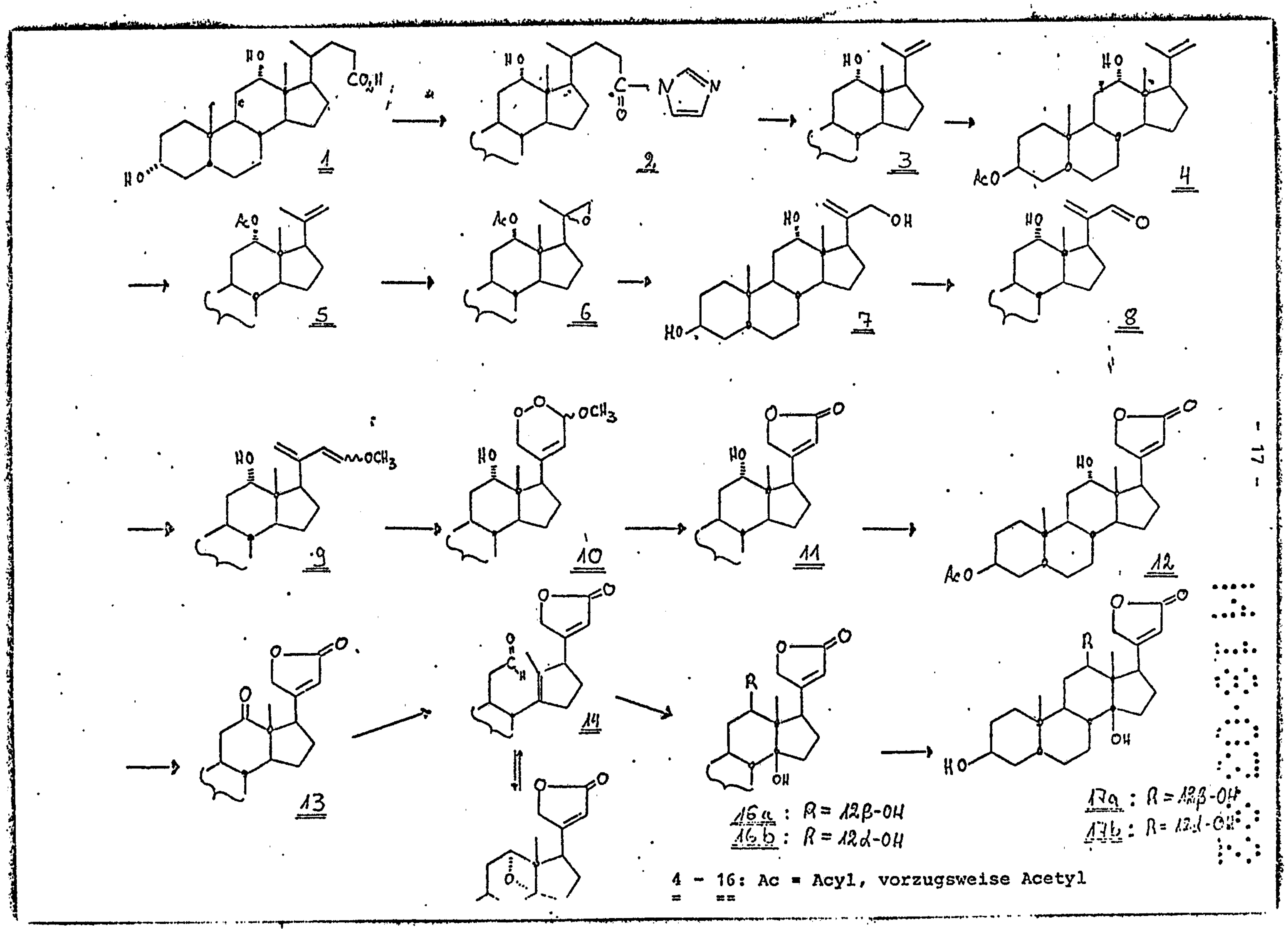
1 2 3 4
5 6 7 8
9 10 11 12
13 14 16a : R = 12β-OH
16b : R = 12α-OH
17a : R = 12β-OH
17b : R = 12α-OH

4 - 16: Ac = Acyl, vorzugsweise Acetyl

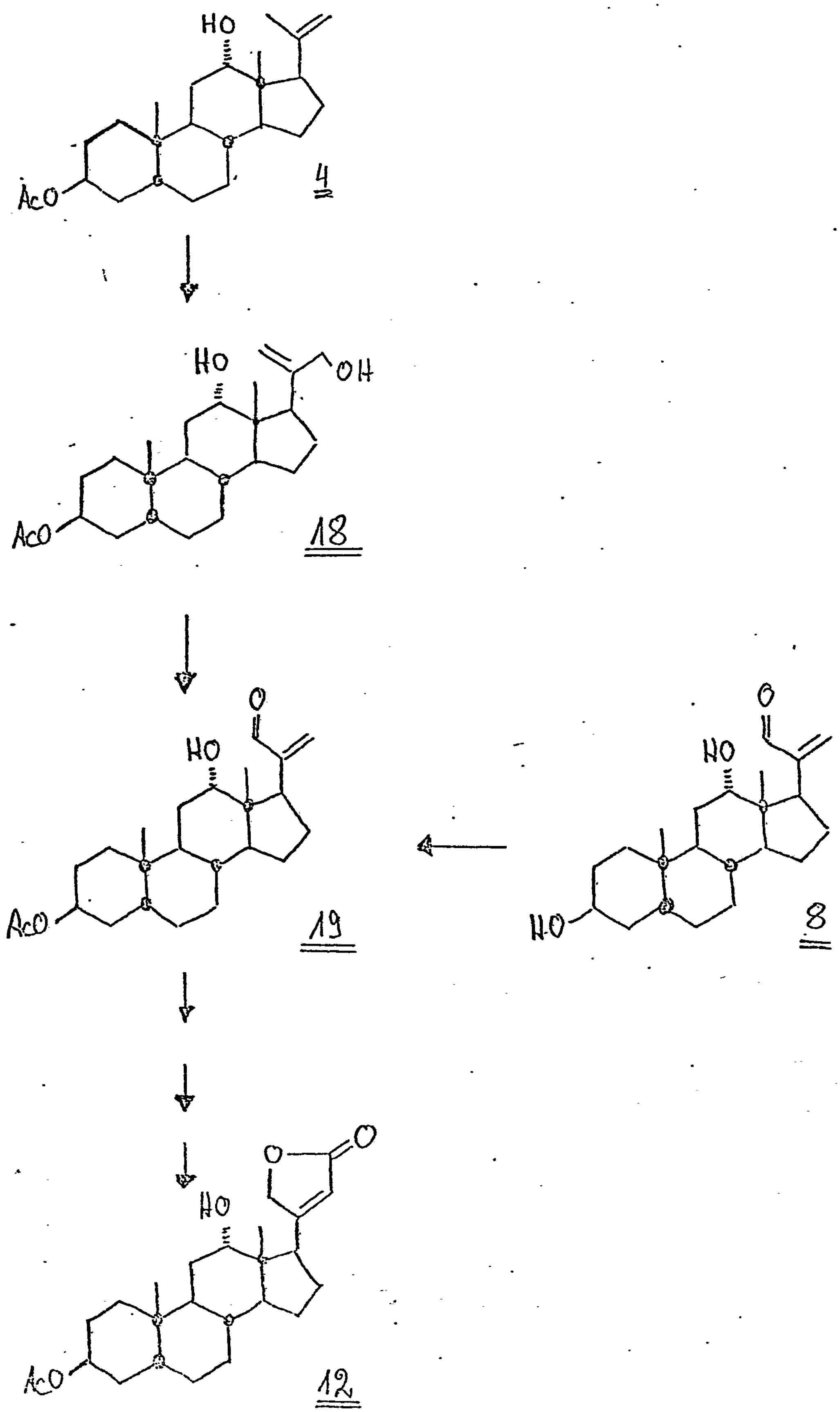

4

18

19

8

12

Patentansprüche

1. Verfahren zur Herstellung des Digoxigenins oder 12-Epidigoxigenins, dadurch gekennzeichnet, daß man ein 3ß-Acetoxy-12-oxo-5ß,14α-card-20(22)-enolid (13) durch Photolyse in den ungesättigten Aldehyd (14) umwandelt, diesen einer intramolekularen Prins-Reaktion unterwirft, wobei die Stereoisomeren 12,14-ß-Diole (16a) und (16b) entstehen und aus diesen die Acetylgruppe in 3-Stellung verseift.

2. Verfahren zur Herstellung des Digoxigenins oder 12-Epidigoxigenins, dadurch gekennzeichnet, daß man
   1) Desoxycholsäure (1) mit Carbonyldiimidazol in das Imidazolid (2) überführt,
   2) dieses zur 20-Methylenverbindung (3) abbaut,
   3) aus dem Produkt (3) durch Konfigurationsumkehr in 3-Position und Acylierung das 3ß-Acyl-Analoge (4) herstellt,
   4) dieses in 12-Stellung acyliert und das 3,12-Bisacetat mit M-Chlorperbenzoesäure zu den Stereoisomeren 20,22-Epoxiden (6) umsetzt,
   5) die Epoxide mit einem Aluminiumalkoholat zur Allylverbindung (7) umlagert, wobei die Acylgruppen verseift werden,
   6) die Allylverbindung (7) zum α,ß-ungesättigten Aldehyd (8) oxidiert,
   7) diesen Aldehyd mit Methoxymethylen-triarylphosphoran zum Gemisch der isomeren Dienoläther (9) umsetzt,
   8) die Dienoläther (9) mit Singulett-Sauerstoff zum 1,2-Dioxan (10) umsetzt,
   9) dieses mit einer organischen Base zum 14α-Cardenolid (11) umlagert
   10) dieses selektiv zur 3ß-Acylverbindung (12) acyliert
   11) diese durch Oxidation in das 12-Keton (13) umwandelt,
   12) dieses durch Photolyse in den ungesättigten Aldehyd (14) umwandelt,

13) diesen einer intramolekularen Prins-Reaktion unterwirft, wobei die Stereoisomeren 12,14ß-Diole (16a) und (16b) entstehen und aus diesen die Acetylgruppe in 3-Stellung verseift.

3. Verfahren nach Anspruch 2, dadurch gekennzeichnet, daß anstelle der Reaktionsschritte 4 bis 10 das 3ß-Acyl-Analoge (4) mit Singulett-Sauerstoff zum 20-Methylen-21-ol-analogen (18) oxidiert und mit dem Produkt entsprechend den Reaktionsstufen 6 bis 10 verfahren wird.

4. Verfahren zur Herstellung von Cardenoliden, dadurch gekennzeichnet, daß man ein 20-Methylen-pregnan-21-al mit Methoxymethylenarylphosphoran zum Gemisch der isomeren 20-Methylen-pregnan-21-methoxy-methylene umsetzt, an diese Singulett-Sauerstoff zum entsprechenden 1,2-Dioxan anlagert und in diesem mit einer organischen Base den Dioxanring zum Butenolidring umlagert.

5. Verbindung der Formel

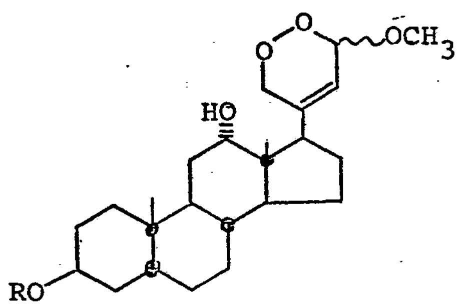

in der R H oder Acyl, vorzugsweise Acetyl ist.

6. Verbindung der Formel

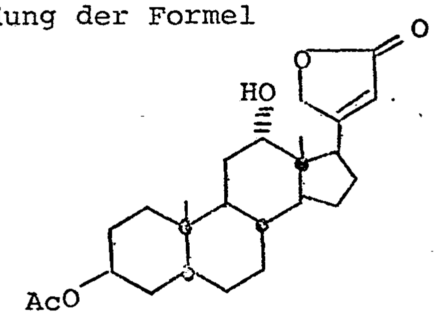

in der Ac Acyl, vorzugsweise Acetyl ist.

7. Verbindung der Formel

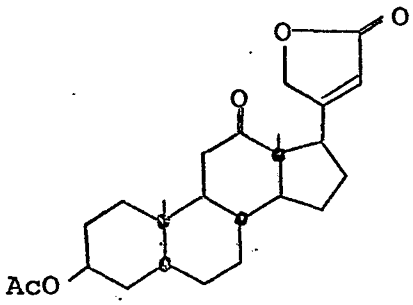

in der Ac Acyl, vorzugsweise Acetyl ist.

Patentansprüche für Österreich

1. Verfahren zur Herstellung des Digoxigenins oder 12-Epidigoxigenins, dadurch gekennzeichnet, daß man ein 3ß-Acetoxy-12-oxo-5ß,14 α-card-20(22)-enolid (13) durch Photolyse in den ungesättigten Aldehyd (14) umwandelt, diesen einer intramolekularen Prins-Reaktion unterwirft, wobei die Stereoisomeren 12,14-ß-Diole (16a) und (16b) entstehen und aus diesen die Acetylgruppe in 3-Stellung verseift.

2. Verfahren zur Herstellung des Digoxigenins oder 12-Epidigoxigenins, dadurch gekennzeichnet, daß man

1) Desoxycholsäure (1) mit Carbonyldiimidazol in das Imidazolid (2) überführt,
2) dieses zur 20-Methylenverbindung (3) abbaut,
3) aus dem Produkt (3) durch Konfigurationsumkehr in 3-Position und Acylierung das 3ß-Acyl-Analoge (4) herstellt,
4) dieses in 12-Stellung acyliert und das 3,12-Bisacetat mit M-Chlorperbenzoesäure zu den Stereoisomeren 20,22-Epoxiden (6) umsetzt,
5) die Epoxide mit einem Aluminiumalkoholat zur Allylverbindung (7) umlagert, wobei die Acylgruppen verseift werden,
6) die Allylverbindung (7) zum α,ß-ungesättigten Aldehyd (8) oxidiert,
7) diesen Aldehyd mit Methoxymethylen-trialkylphosphoran zum Gemisch der isomeren Dienoläther (9) umsetzt,
8) die Dienoläther (9) mit Singulett-Sauerstoff zum 1,2-Dioxan (10) umsetzt,
9) dieses mit einer organischen Base zum 14α-Cardenolid (11) umlagert
10) dieses selektiv zur 3ß-Acylverbindung (12) acyliert,
11) diese durch Oxidation in das 12-Keton (13) umwandelt,

12) dieses durch Photolyse in den ungesättigten Aldehyd (14) umwandelt,

13) diesen einer intramolekularen Prins-Reaktion unterwirft, wobei die Stereoisomeren 12,14ß-Diole (16a) und (16b) entstehen und aus diesen die Acetylgruppe in 3-Stellung verseift.

3. Verfahren nach Anspruch 2, dadurch gekennzeichnet, daß anstelle der Reaktionsschritte 4 bis 10 das 3ß-Acyl-Analoge (4) mit Singulett-Sauerstoff zum 20-Methylen-21-ol-analogen (18) oxidiert und mit dem Produkt entsprechend den Reaktionsstufen 6 bis 10 verfahren wird.

4. Verfahren zur Herstellung von Cardenoliden, dadurch gekennzeichnet, daß man ein 20-Methylen-pregnan-21-al mit Methoxymethylen-triarylphosphoran zum Gemisch der isomeren 20-Methylen-pregnan-21-methoxy-methylene umsetzt, an diese Singulett-Sauerstoff zum entsprechenden 1,2-Dioxan anlagert und in diesem mit einer organischen Base den Dioxanring zum Butenolidring umlagert.

5. Verfahren zur Herstellung einer Verbindung der Formel

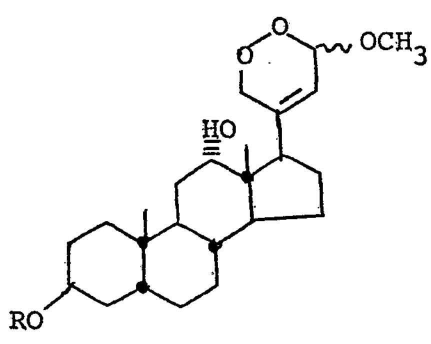

in der R H oder Acyl, vorzugsweise Acetyl ist, dadurch gekennzeichnet, daß der entsprechende Aldehyd mit Methoxymethylen-triarylphosphoran zum Gemisch der isomeren Dienoläther umgesetzt und diese mit Singulett-Sauerstoff umgesetzt werden.

6. Verfahren zur Herstellung einer Verbindung der Formel

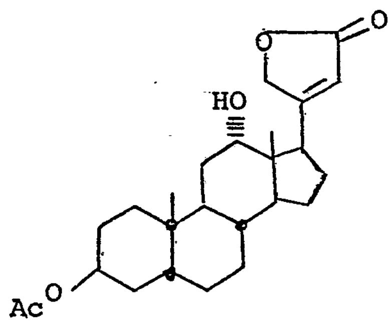

in der Ac Acyl, vorzugsweise Acetyl ist, dadurch gekennzeichnet, daß eine Verbindung gemäß Anspruch 5, in der R für Acyl steht, mit einer organischen Base umgelagert wird.

7. Verfahren zur Herstellung einer Verbindung der Formel

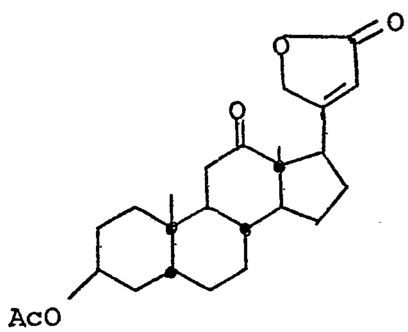

in der Ac Acyl, vorzugsweise Acetyl ist, dadurch gekennzeichnet, daß die entsprechende α-Hydroxy-Verbindung oxidiert.